# EUROPÄISCHE PATENTANMELDUNG

(11) **EP 4 686 459 A2**
(43) Veröffentlichungstag der Anmeldung: **04.02.2026**
(21) Anmeldenummer: 25210178.7
(22) Anmeldetag: 07.04.2025
(51) Int. Cl.: A61F 7/00

(54) **KOMPAKTE, MULTIFUNKTIONALE VORRICHTUNG ZUR BEHANDLUNG VON INSEKTENSTICHEN**

(30) Priorität: 25.07.2024 DE 202024104190 U; 02.08.2024 DE 202024104366 U
(62) Teilanmeldung aus: 25168842.0
(71) Anmelder: Horst Dittmann e.K., 97727 Fuchsstadt (DE)
(72) Erfinder: Dittmann, Horst, 97727 Fuchsstadt (DE)
(74) Vertreter: Scharfenberger, Burkhard

(57) **Zusammenfassung**

Vorliegende Erfindung betrifft eine multifunktionale Vorrichtung, welche unter anderem zur Behandlung von Insektenstichen und -bissen einsetzbar ist und umfasst: ein Gehäuse (2), eine Heizplatte in einer Behandlungsfläche (23) am vorderen Gehäuseende, ein oder mehrere Leuchtmittel (4, 4a, 4b), welche/s in einem oder mehreren Leuchtmittelbereichen (24, 24a, 24b) angeordnet ist, eine elektrische Energiequelle (5), mittels der die Heizplatte (3) und das Leuchtmittel (4) mit elektrischer Energie versorgbar ist, und eine Steuerelektronik, welche dazu vorbereitet ist, die Heizplatte (3) mit der Energiequelle (5) entnommener elektrischer Energie zu versorgen, und die Heizplatte (3) dabei für eine gewünschte Behandlungsdauer auf eine gewünschte Behandlungstemperatur aufzuheizen, wobei die Steuerelektronik auf einer Platine (6) untergebracht ist, welche in einer Mittenebene des Gehäuses angeordnet ist

## Beschreibung

Vorliegende Erfindung betrifft eine multifunktionale Vorrichtung zur Behandlung von Insektenstichen umfassend ein Gehäuse, ein in einer Behandlungsfläche des Gehäuses integrierte Heizplatte sowie ein Leuchtmittel, das in ein Leuchtmittelbereich des Gehäuses angeordnet ist.

Zur Behandlung von Insektenstichen und -bissen, oder anderen mit Histaminausschüttung verbunden lokalen Hautreizungen sind Wärmebehandlungsvorrichtungen bekannt, mit der die juckende Stelle kurzzeitig auf eine Temperatur erhitzt werden kann, die ausreichend hoch ist, das Histamin thermisch zu zersetzen, aber niedrig genug, dass dabei keine Hautverbrennungen zu verursacht werden. Derartige Vorrichtungen umfassen dazu eine, üblicherweise im Bereich des vorderen Endes eines Gehäuses angeordnete Heizplatte, die auch für eine gewünscht Behandlungsdauer auf eine gewünschte Behandlungstemperatur erhitzt wird. Die Energie hierfür wird von einem elektrischen Energiespeicher, typischerweise einer Batterie, die Teil der Vorrichtung oder extern untergebracht sein kann, bereitgestellt und durch eine Steuerelektronik der Vorrichtung der Heizplatte zugeleitet. Eine Vorrichtung mit diesem Grundsätzlichen Aufbau wurde bereits in der europäischen Patentschrift EP 1231875B1 vorgestellt.

Für dieses Grundkonzept wurden verschiedenen Verbesserungen vorgeschlagen, um die Funktionalität und Sicherheit derartiger Vorrichtungen zu erhöhen. So stellt beispielsweise die Offenlegungsschrift EP 3308752A1 einen Insektenstichheiler des vorgenannten Aufbaus vor, bei dem ein hardwareimplementierter Temperaturwächter eingesetzt wird, um bei Auftreten eines Steuerungsdefekts ein unkontrolliertes Durchheizen der Heizplatte zu verhindern. Die Offenlegungsschrift DE 10 2017 006 994A1 zeigt ein sehr kompaktes Insektenstichgerät ohne eigene Energiequelle, welches dafür über eine Schnittstelle verfügt, mit der es an ein Mobilgerät, etwa ein Smartphone oder Tablet, koppelbar ist, so dass es durch dieses steuerbar und - vor allem - mit elektrischer Energie versorgbar ist. Eine ähnliche Vorrichtung wird in der deutschen Gebrauchsmusterschrift DE 20 2019 005553U1 vorgestellt. Ein zweiteiliger Insektenstichheiler umfassend einen Vorderteil mit einer Schnittstelle zu einem Mobilgerät und einen hinteren Teil mit einer Energiequelle ist in der WO 2019/234172A1 offenbart. Die Offenlegungsschrift WO 2020/049070A1 schlägt einen Insektenstichheiler mit verbesserter Sicherheit vor, bei dem die Heizplatte nur dann mit Spannung beaufschlagt, also beheizt, wird wenn ein Kontaktsensor im Behandlungsbereich feststellt, dass die Heizplatte Hautkontakt hat. Die Offenlegungsschrift EP 3269340A1 zeigt einen Insektenstichheiler, in dem verschiedene Parameter des Gerätes wie Größe und Ausgestaltung der Behandlungsfläche, oder auch Anteil der Heizelemente optimiert sind. In der Offenlegungsschrift WO 2018/0157264A1 wird ein Insektenstichheiler vorgestellt, der zusätzlich zur Wärmebehandlung auch eine Behandlung mit einem Medikament vornehmen kann, wobei dieses in eine Medikamentenkammer füllbar ist. Alle vorgenannten Schriften verbessern verschiedene Eigenschaften von Insektenstichheilern, verbreitern jedoch das Einsatzspektrum derartiger Vorrichtungen nicht.

Über das bisher bekannte hinaus geht jedoch die Offenlegungsschrift WO 2020/152239A1, in welcher ein Insektenstichheiler des grundsätzlich bekannten Aufbaus vorgeschlagen wird, der jedoch außer einem Interface zu einem Mobilgerät auch über einen Speicher für verschiedene Behandlungsprogramme verfügt, wobei das Gerät mittels des angekoppelten Mobilgeräts gesteuert wird. Dies ermöglicht es, ein für die jeweilige Situation passendes Behandlungsprogramm auszuwählen, beispielsweise für größere, juckende Stellen ein Programm, bei dem Behandlungsdauer und/oder Behandlungstemperatur gegenüber einem Normalprogramm erhöht sind, oder aber für empfindlichere Hautpartien und/oder Personen Behandlungsprogramme bereitzustellen, welche eine kürzere Behandlungsdauer und/oder niedrigere Behandlungstemperatur haben. Das Einsatzspektrum des Insektenstichheilers ist aber auch bei dieser Schrift in anderer Hinsicht noch begrenzt. So ist beispielsweise eine Anwendung der Vorrichtung im Dunkeln ohne externe Lichtquelle das Auffinden und zielgenaue aufsetzen der dortigen Vorrichtung auf eine juckende Hautstelle rein nach Tastgefühl sehr schwierig und auch zumeist nur durch die betroffene Person selbst möglich.

Eine Lösung dieses Problems wird in den Schriften KR 101912442B1, DE 10 2021 123 177 A1 und CN 211C433629U vorgeschlagen, welche jeweils einen Insektenstichheiler um ein Leuchtmittel ergänzen, mittels dem die zu behandelnde Stelle leuchtbar ist. Der Nachteil bei den dortigen Lösungen ist jedoch, dass das Leuchtmittel im unmittelbaren Zusammenhang mit der Heizplatte steht, und teilweise nur zusammen mit dieser aktivierbar ist. Weiterhin sind die vorgeschlagenen Leuchtmittel auch von geringer Leuchtkraft, so dass lediglich die Beleuchtung des unmittelbaren Behandlungsbereich möglich ist.

Vor diesem Hintergrund hat sich vorliegende Erfindung die Aufgabe gestellt, eine Wärmebehandlungsvorrichtung für Insektenstiche dahingehend zu verbessern, dass sie ein weites Einsatzspektrum und vielfältigerer Einsatzmöglichkeiten aufweist als bekannte Vorrichtungen.

Eine weitere Aufgabe der Erfindung ist es, eine besonders kompakte und platzsparende Vorrichtung zu schaffen.

Gelöst werden diese und andere Aufgaben durch multifunktionale Wärmebehandlungsvorrichtungen nach dem unabhängigen Anspruch 1, welche in den Unteransprüchen vorteilhaft weitergebildet werden.

Die erfindungsgemäßen multifunktionalen Wärmebehandlungsvorrichtungen umfasst dabei in grundsätzlich bekannter Art ein Gehäuse, eine Heizplatte, welche in einer Behandlungsfläche angeordnet ist, die an einem vorderen Ende des Gehäuses liegt, eine elektrische Energiequelle und eine Steuerelektronik zur kontrollierten Energieversorgung der Heizplatte, um diese für eine Behandlungsdauer auf eine Behandlungstemperatur aufzuheizen, sowie ein Leuchtmittel, welches in einem Leuchtmittelbereich des Gehäuses angeordnet ist und ebenfalls seine Energie aus der Energiequelle bezieht. Das Leuchtmittel bzw. der Leuchtmittelbereich sind dabei nicht notwendig in dem Bereich oder in der Nähe der Behandlungsfläche angeordnet.

Wesentlich für einen ersten Aspekt der Erfindung ist, dass das Leuchtmittel unabhängig von der Heizplatte aktivierbar und deaktivierbar ist, so dass die Beleuchtungsfunktion und die Wärmebehandlungsfunktion der multifunktionalen Vorrichtung separat und unabhängig voneinander einsetzbar sind. Hierdurch kann die Vorrichtung je nach Wunsch zur Behandlung eines Insektenstichs oder -bisses oder einer anderen lokalen, juckenden Hautreizung eingesetzt werden oder sie wird in der Art einer Taschenlampe zur Beleuchtung der Umgebung verwendet. Entsprechend im Stand der Technik bekannter Wärmebehandlungsvorrichtungen können beide Funktionen auch kombiniert werden, um eine im Dunklen schwer auffindbare oder gezielt behandelbare juckende Hautstelle zu versorgen.

Ein zweiter Aspekt der Erfindung betrifft den inneren Aufbau der Vorrichtung und die Ausgestaltung und Anordnung der dortigen Komponenten. Nach diesem Aspekt ist die Steuerelektronik auf einer Platine untergebracht, welche wiederum in oder Nahe einer Mittenebene des Gehäuseinnenraums angeordnet ist. Die Platine erstreckt sich dabei bevorzugt durch den gesamten Gehäuseinnenraum von einem vorderen Ende bis zu einem hinteren Ende. Hierdurch steht vorteilhafterweise ein Halbraum oder sogar etwas mehr des Gehäuseinnenraums zur Unterbringung des Energiespeichers zur Verfügung. Weiterhin können die in der Nähe der Gehäuseenden anzuordnenden Komponenten der Vorrichtung, wie die Heizplatte und das oder die Leuchtmittel an den Stirnseiten der Platine angeordnet werden.

Leuchtmittel und/oder Heizplatte können unmittelbar auf der Platine untergebracht werden. Bevorzugt sind aber die Heizplatte und ein in ihrer Nähe angeordnetes, als Suchlicht dienendes Leuchtmittel auf einem separaten Heizplattenträgerelement untergebracht. Dies vereinfacht den Zusammenbau der Vorrichtung und verbessert die thermische Entkopplung des Heizelements und der teilweise hitzeempfindlichen Elektronikkomponenten der Steuerelektronik. Das Heizplattenträgerelement umfasst bevorzugt einen in einer vorderen Gehäuseöffnung angeordneten Trägerteil und eine davon abgewinkelte Kontaktschiene, welche zu einem Pol des elektrischen Energiespeichers geführt ist.

Die erfindungsgemäßen Merkmale erlauben die Bereitstellung mehrerer Funktionen, zumindest einer Insektenstichbehandlungsfunktion und einer Beleuchtungsfunktion, in einem Gerät von besonders kompaktem Aufbau.

Weitere vorteilhafte Weiterbildungen, welche einzeln oder in Kombination realisierbar sind, sofern sie sich nicht gegenseitig offensichtlich ausschließen, sind in den Unteransprüchen beansprucht und werden nachfolgend im Einzelnen vorgestellt.

Bei der erfindungsgemäßen Vorrichtung kann das oder mindestens eines der Leuchtmittel an einer Stirnseite der sich in einer Mittenebene des Gehäuseinnenraums erstreckenden Platine angeordnet sein und die Lichtemissionsrichtung des Leuchtmittels von der Platine weg weisen.

Der Energiespeicher kann den zwischen der Platine und einer Innenwandung des Gehäuses verbleibenden Raum auf einer Seite der Platine nahezu vollständig ausfüllen, um die Speichergröße und damit die zum Aufheizen der Heizplatte und/oder für das oder die Leuchtmittel zur Verfügung stehende elektrische Energie zu maximieren. Bei der bevorzugten im Wesentlichen kapsel- oder sphärozylindrischen Gehäuseform, verbleibt beispielsweise für die Unterbringung des Energiespeichers auf einer Seite der Platine im Wesentlichen der halbzylindrischer Raum im Gehäusemittelteil sowie ein Teil der daran anschließenden Viertelkugeln.

Der Leuchtmittelbereich, in dem das (mindestens eine) Leuchtmittel der erfindungsgemäßen Vorrichtung angeordnet ist, kann an einem dem vorderen Ende gegenüberliegenden hinteren Ende des Gehäuses befindlich sein. Alternativ oder zusätzlich ist der bzw. einer der Leuchtmittelbereich/e an dem vorderen Gehäuseende gelegen, an dem sich auch die Behandlungsfläche mit der Heizplatte befindet. In diesem Fall ist es insbesondere bevorzugt, dass der Leuchtmittelbereich und das oder die darin angeordneten Leuchtmittel die Behandlungsfläche ringförmig umgeben. Beispielsweise kann der Leuchtmittelbereich mehrere, die Heizplatte konzentrisch umgebende Leuchtdioden als Leuchtmittel umfassen. Das Leuchtmittel kann an eine Stirnseite der Platine, insbesondere einer hinteren Stirnseite im Bereich des hinteren Gehäuseendes angeordnet sei und mit seiner Hauptlichtemissionsrichtung von der Platine weg weisen.

Das oder eines der Leuchtmittel und die Heizplatte können nebeneinander aber beabstandet zueinander auf einem Heizplattenträgerelement untergebracht sein. Dabei hat das Leuchtmittel insbesondere einen Abstand zu dem (Rand der) Heizplatte, welcher in etwa seinen Abmessungen entspricht, beispielswese in einem Abstand von 0,5 - 2 mm. Die Anordnung des Leuchtmittels neben der Heizplatte also an ihrer schmalen Seite verringert die thermische Kopplung zwischen beiden Komponenten. Die Anforderungen an die thermische Isolation des Leuchtmittels von der Heizplatte sind zwar dadurch reduziert, dass letztere bevorzugt auf maximal zwischen 50 und 55°Grad Celsius aufgeheizt wird. Dennoch ist eine thermische Isolierung zur Verlängerung der Lebensdauer des Leuchtmittels vorteilhaft.

Zwischen Heizplatte und Heizplattenträgerelement bleibt mit Ausnahme der elektrischen Zuleitungen zur Heizplatte in manchen Ausführungen ein Luftspalt, so dass nahezu keine Kontaktwärmeübertragung von der Heizplatte auf das Heizplattenträgerelement stattfindet.

Die Vorrichtung kann eine transparente oder zumindest durchscheinende Abdeckung mit einer rückwärtigen Ausnehmung für das Leuchtmittel umfassen. Die Abdeckung ist bevorzugt als ein die Heizplatte umgebendes Element realisiert, welches einen diffusiven Lichtleiter umfasst, der das Licht eines oder mehrerer Leuchtmittel gleichmäßig um sie verteilt abgibt. Das in der rückwärtigen Ausnehmung aufgenommene Leuchtmittel wird durch einen Teilbereich der Abdeckung von der Heizplatte separiert, so dass keine direkte Sichtlinie zwischen dem Leuchtmittel und der Heizplatte besteht. Dies verhindert ein radiative Aufheizung des Leuchtmittels durch die von der heißen Heizplatte abgegebene Wärmestrahlung.

Ist der Leuchtmittelbereich bzw. einer der Leuchtmittelbereiche am hinteren Gehäuseende gelegen, kann ein einzelnes, lichtstarkes Leuchtmittel vorhanden sein, oder es können auch mehrere Leuchtmittel zum Einsatz, deren Lichtstrom und ggf. -farbe sich zu einer Gesamtlichtstrom und einer Gesamtfarbe addiert. Das oder die Leuchtmittel am hinteren Gehäuseende ist bevorzugt eine oder mehrere LEDs. Der von dem/n Leuchtmittel/n am hinteren Gehäuseende abgebbare Lichtstrom beträgt bevorzugt mindestens 100, noch mehr bevorzugt zwischen 400 und 1000 Lumen.

Die erfindungsgemäße Vorrichtung kann ein Bedienelement umfassen mittels dessen das Leuchtmittel ein- und ausschaltbar ist. Bei Ausführungsformen mit mehr als einem Leuchtmittelbereich, etwa zwei Leuchtmittelbereichen am vorderen und hinteren Gehäuseende, kann auch je ein Bedienelement pro Leuchtmittel vorhanden sein. Das bzw. die Bedienelement/e ist/sind insbesondere ein Knopf oder ein Schalter.

Die erfindungsgemäße Vorrichtung kann eine Kappe umfassen, welche das vordere Gehäuseende bedeckt. Die Kappe kann mittels eines Bajonettverschlusses an dem Gehäuse verriegelbar sein.

Die elektrische Energiequelle der erfindungsgemäßen Vorrichtung ist bevorzugt ein Energiespeicher, insbesondere eine (wiederaufladbare) Batterie, wie etwa eine wiederaufladbare Lithium-Ionen-Batterie. Diese kann herausnehmbar und extern aufladbar gestaltet sein. Alternativ umfasst die Vorrichtung eine Ladebuchse, mittels der bei Anschluss einer geeigneten Spannungsquelle der Energiespeicher, aufladbar ist. Die Ladebuchse kann in einer von dem vorderen Gehäuseende und/oder dem hinteren Gehäuseende verschiedenen Seite des Gehäuses angeordnet sein, etwa einer Längsseite des Gehäuses.

Das Gehäuse der erfindungsgemäßen Vorrichtung ist bevorzugt länglich, und insbesondere zylindrisch oder sphärozylindrisch (kapselförmig) mit einem zylindrischen Mittelteil, an welchen sich in etwa halbkugelige Endbereiche anschließen.

Weiterhin hat das Gehäuse, gegebenenfalls mit aufgesetzter Kappe, bevorzugt eine Länge von zwischen 50 und 100 mm, insbesondere ca. 65 mm und einen Durchmesser von zwischen 20 und 40, insbesondere 25 mm. Die Kappe hat bevorzugt eine Höhe von zwischen 1/3 und 4/5, insbesondere von circa 2/3 der Gehäuselänge. Das Gehäuse kann wasserdicht oder zumindest spritzwassergeschützt sein, was die vielfältige Einsetzbarkeit der erfindungsgemäßen Vorrichtung weiter steigert.

Die erfindungsgemäße Vorrichtung kann weiterhin eine Steuerelektronik umfassen, welche dazu vorbereitet ist, die Heizplatte mit der Energiequelle entnommener elektrischer Energie zu versorgen, und die Heizplatte dabei für eine gewünschte Behandlungsdauer auf eine gewünschte Behandlungstemperatur aufzuheizen.

Die Behandlungsdauer liegt dabei bevorzugt zwischen 2 Sekunden und 10 Sekunden, insbesondere zwischen 3 Sekunden und 6 Sekunden beträgt, und die Behandlungstemperatur zwischen 46 Grad Celsius und 54 Grad Celsius, insbesondere zwischen 48 Grad Celsius und 51 Grad Celsius. Besonders bevorzugt beträgt die Behandlungsdauer 3 Sekunden bei einer Behandlungstemperatur von 48 Grad Celsius oder 51 Grad Celsius und/oder die Behandlungsdauer beträgt 6 Sekunden bei einer Behandlungstemperatur von 51 Grad Celsius.

In manchen Ausführungsformen der Erfindung umfasst die Steuerelektronik einen Programmspeicher, in dem zwei oder mehr Behandlungsprogramme abgespeichert sind. Dabei weist die Vorrichtung weiterhin ein Bedienmittel auf, insbesondere ein Programmwahlknopf oder - taster, mittels dessen durch einen Benutzer der Vorrichtung eines der im Programmspeicher abgespeicherten Programme auswählbar und/oder ausführbar ist. Dabei ist die Steuerelektronik bevorzugt so eingerichtet, dass ein Programm nach dem Auswählen automatisch ausgeführt wird.

Die im Programmspeicher abgespeicherten Behandlungsprogramme können ein erstes, beispielsweise für Kinder geeignetes, Behandlungsprogramm umfassen, bei dem die Behandlungsdauer von 3 Sekunden und die Behandlungstemperatur von 48 Grad Celsius beträgt. Die Behandlungsprogramme können weiterhin auch ein zweites für empfindliche Erwachsene oder kleinere Stiche oder -bisse geeignetes Behandlungsprogramm umfassen, in dem die Behandlungsdauer 3 Sekunden und die Behandlungstemperatur von 51 Grad Celsius beträgt. Die Behandlungsprogramme können weiterhin auch ein drittes, für größere, stärker geschwollene Stich- oder Bissstellen geeignetes Behandlungsprogramm umfassen, in dem die Behandlungsdauer 6 Sekunden und die Behandlungstemperatur von 51 Grad Celsius beträgt.

Die erfindungsgemäße Vorrichtung umfasst weiterhin bevorzugt eine An-/Austaste und ist dazu vorbereitet von einem ausgeschalteten Zustand in einen Bereitschaftszustand überzugehen, wenn die An-/Austaste für mindestens eine Aktivierungszeitdauer, insbesondere zwischen 2 Sekunden und 4 Sekunden, bevorzugt 3 Sekunden, gedrückt wird. Weiterhin ist die Vorrichtung dazu vorbereitet, aus dem Bereitschaftszustand in einen aktiven Zustand, in dem die Heizplatte gemäß einem der abgespeicherten Programme aktiviert ist, überzugehen, wenn die An-/Austaste ein oder mehrmals kurz, d.h. für eine Sekunde oder weniger, gedrückt wird, wobei insbesondere eine Anzahl der An-/Aus-Tastendrücke entscheidet, welches der mindestens zwei abgespeicherten Behandlungsprogramme ausgeführt wird. Die An-/Austaste kann unterhalb der Kappe angeordnet sein.

Die erfindungsgemäße Vorrichtung ist in bevorzugten Ausführungsformen in der Lage dem Benutzer eine Rückmeldung über den derzeitigen Betriebszustand und/oder das derzeit auswählte und/oder ausgeführte Programm zu geben. Hierzu verfügt sie über eine Zustandsanzeige, bevorzugt in der Form eines hinter der An-/Austaste angeordneten Leuchtmittels, welches im ausgestalteten Zustand nicht leuchtet und im Bereitschaftszustand und im aktiven Zustand leuchtet.

Besonders bevorzugt kann dieses Zustandsanzeigeleuchtmittel farbig leuchten, insbesondere umfasst es farbige LEDs. Es kann dazu vorbereitet sein, in einer ersten Farbe zu leuchten, insbesondere in grüner Farbe, wenn sich die Vorrichtung im Bereitschaftszustand befindet, und in einer von der ersten Farbe verschiedenen zweiten Farbe zu leuchten, wenn sich die Vorrichtung im aktiven Zustand befindet, in dem die Heizplatte beheizt ist.

Diese zweite Farbe ist insbesondere bevorzugt im Falle des oben genannten ersten Behandlungsprogramms Blau, im Falle des zweiten Behandlungsprogramms Weiß und im Falle des dritten Behandlungsprogramms Violett.

Die erfindungsgemäße Vorrichtung umfasst bevorzugt zwei Leuchtmittel, wobei ein zweites Leuchtmittel in einem zweiten Leuchtmittelbereich an einem dem vorderen Ende gegenüberliegenden hinteren Gehäuseende angeordnet ist und ein erstes Leuchtmittel in einem ersten Leuchtmittelbereich an dem vorderen Gehäuseende angeordnet ist. Der erste Leuchtmittelbereich kann die Behandlungsfläche insbesondere ringförmig umschließen und mehrere, ring- oder kranzförmig um die Heizplatte angeordnete Leuchtmittel, beispielsweise LEDs, aufweisen.

Bei diesen Ausführungen der Erfindung hat das zweite Leuchtmittel am hinteren Gehäuseende bevorzugt eine größere Leuchtkraft als das erste Leuchtmittel am vorderen. Insbesondere hat das zweite Leuchtmittel einen maximalen Lichtstrom von zwischen 50 und 1000 Lumen, bevorzugt zwischen 400 und 1000 Lumen, das erste Leuchtmittel hat bevorzugt einen maximalen Lichtstrom von 1 bis 100 Lumen, bevorzugt zwischen 5 und 40 Lumen.

Es ist bevorzugt, dass das zweite Leuchtmittel weißes Licht emittiert wohingegen das erste Leuchtmittel wahlweise weißes Licht oder farbiges Licht emittieren kann, insbesondere rotes, blaues oder grünes Licht. Die Farbwahl kann über eines der Bedienelemente erfolgen.

Die Steuerelektronik ist bevorzugt dazu vorbereitet ist, dass zweite Leuchtmittel automatisch zu aktivieren, wenn die Vorrichtung im Bereitschaftszustand und/oder im aktiven Zustand ist. Weiterhin kann die erfindungsgemäße Vorrichtung aber auch ein weiteres Bedienelement umfassen, mittels dessen das zweite Leuchtmittel unabhängig vom Betriebszustand der Vorrichtung aktivierbar und deaktivierbar ist.

Weitere Merkmale, Eigenschaften und Vorteile vorliegender Erfindung ergeben sich aus den im Folgenden unter Bezugnahme auf die Figuren vorgestellten bevorzugten Ausführungsbeispiele, welche vorliegende Erfindung lediglich illustrieren und in keiner Weise in ihrer Allgemeinheit einschränken sollen.

Es zeigen:
- Figur 1A:: Eine perspektivische Ansicht einer ersten Ausführungsform einer erfindungsgemäßen multifunktionalen Wärmebehandlungsvorrichtung mit aufgesetzter Kappe und Blick auf ein hinteres Ende, in welches ein starkes Leuchtmittel integriert ist.
- Figur 1B:: Eine perspektivische Ansicht der Ausführungsform der Figur 1A ohne Kappe mit Blick auf das dadurch freigegebene vordere Ende mit integrierter Heizplatte und dem die Heizplatte umgebenden Suchlicht.
- Figur 1C:: Eine Seitenansicht der Ausführungsform der vorhergehenden Figuren mit Kappe.
- Figur 1D:: Eine zweite Seitenansicht der Ausführungsform der vorhergehenden Figuren ohne Kappe aus einer Perspektive, in der die Vorrichtung im Vergleich zu Figur 1C um +90° um die Längsachse(Hochachse) gedreht ist.
- Figur 1E:: Eine zweite Seitenansicht der Ausführungsform der vorhergehenden Figuren ohne Kappe aus der Perspektive der Figur 1C.
- Figur 1F:: Eine perspektivische Ansicht der Ausführungsform der erfindungsgemäßen Wärmebehandlungsvorrichtung nach den vorhergehenden Figuren mit abgenommener Kappe, wobei Kappe und der Rest der Vorrichtung separat dargestellt sind.
- Figur 2A:: Eine perspektivische Ansicht der Ausführungsform der erfindungsgemäßen Wärmebehandlungsvorrichtung der Figuren 1 mit aufgesetzter Kappe und einem and die Kappe integriertem Tragemittel in Form einer Tragkordel.
- Figur 2B:: Eine perspektivische Ansicht des vorderen Endes einer erfindungsgemäßen Vorrichtung gemäß den vorliegenden Figuren mit aufgesetzter Kappe und einer an der Kappe befestigten weiteren Haltemittel in Form eines Karabinerhakens.
- Figur 3A: Eine perspektivische Explosionsdarstellung der Ausführungsform der erfindungsgemäßen Vorrichtung nach den vorhergehenden Figuren, aus der ihre Komponenten und ihre qualitative Anordnung ersichtlich sind.
- Figur 3B:: Eine perspektivische Ansicht des Heizplattenträgerelements der bevorzugten Ausführungsform.
- Figur 3C:: Perspektivische Vorderansicht der Leuchtmittelabdeckung mit Lichtleiterfunktion.
- Figur 3D:: Perspektivische Rückansicht der Leuchtmittelabdeckung.
- Figur 3E:: Längsschnitt durch die erfindungsgemäße Vorrichtung gemäß der bevorzugten Ausführungsform im zusammengesetzten Zustand.

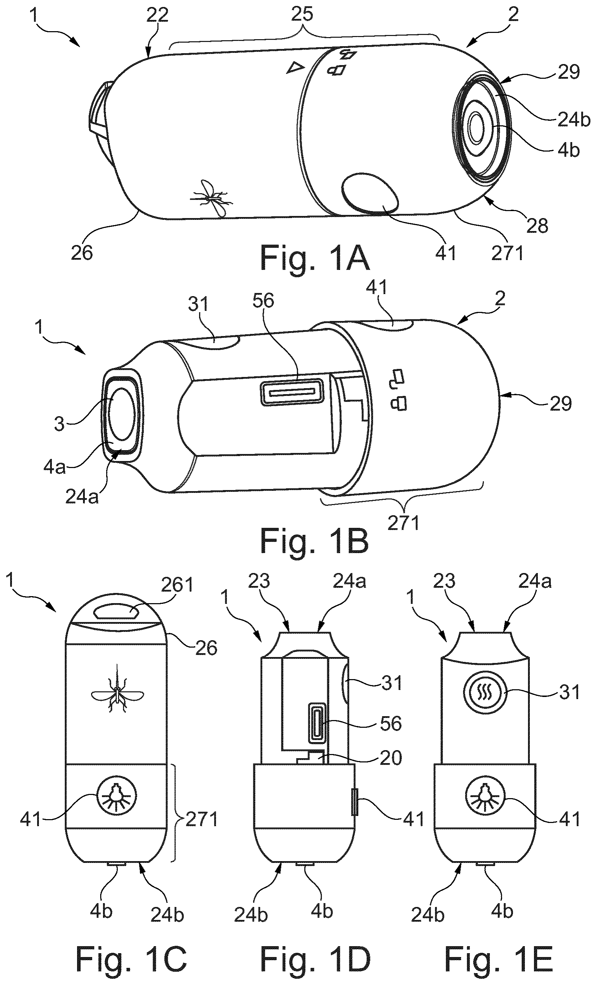

Die **Figuren 1A** - **1F** zeigen verschiedene Ansichten einer bevorzugten Ausführungsform der erfindungsgemäßen Wärmebehandlungsvorrichtung, die sich durch ihre Funktionalität bei kompakten Abmessungen auszeichnet.

Die Wärmebehandlungsvorrichtung 1 umfasst dabei das Gehäuse 2, in dem eine Heizplatte 3 zur Durchführung einer Wärmebehandlung einer juckenden Hautstelle und zwei Leuchtmittel 4 integriert sind. Das Gehäuse umfasst dabei die Kappe 26 und das Hauptteil 27 mit Griffteil 271, wobei erstere mittels eines bajonettartigen Verschlusses umfassend die Kerbe 20 an dem Hauptteil 27 verriegelbar ist, um ein ungewolltes Lösen zu verhindern.

Die kreisrunde Heizplatte 3 befindet sich in dem von der Kappe 26 überdeckten bzw. überdeckbaren vorderen Gehäuseende 21 in der Mitte der in etwa quadratischen Behandlungsfläche 22 integriert. Die Heizplatte 3 kann von der im Inneren des Gehäuses 2 untergebrachten Steuerelektronik mit einer Batterie (beides nicht dargestellt) entnommener elektrischer Energie versorgt werden, um die Heizplatte 3 für eine gewisse, gewünschte Behandlungsdauer auf eine gewünschte Temperatur aufzuheizen. Die Vorrichtung 1 kann dabei Temperatursensoren umfassen, mittels derer die Steuerelektronik die gegenwärtige (Ist-)Temperatur der Heizplatte 3 messen kann, um diese auf den gewünschten Sollwert einzuregeln.

Die Heizplatte 3 ist von einem Leuchtmittelbereich 24a umgeben, in dem ein erstes Leuchtmittel 4a in Form mehrerer, die Heizplatte 3 umgebender, LEDs untergebracht ist, welche als Suchlicht zum Beleuchten der Behandlungsstelle im Dunkeln dienen. Am gegenüberliegenden hinteren Gehäuseende 29 ist in einem zweiten Leuchtmittelbereich 24b ein zweites Leuchtmittel 4b in Form einer oder mehrere LEDs untergebracht. Da es nur eine Beleuchtung im Nah- und Nächstbereich von unterhalb ca. 10-15 cm Abstand dient, kann das Suchlicht 4a vergleichsweise leuchtschwach sein, um Energie zu sparen. Das zweite Leuchtmittel 4b ist hingegen deutlich leuchtstärker und erlaubt es der Vorrichtung 1, die Funktion einer Taschenlampe zu erfüllen. Vorteilhafterweise ist das hintere Gehäuseende 29 nicht durch die Kappe 26 bedeckt, so dass das zweite Leuchtmittel 4b unabhängig von der Kappe 26 eingesetzt werden kann. Der maximale Lichtstrom des Suchlichts 4a beträgt 5 - 20 Lumen, bevorzugt in etwa 10 Lumen, was bei einer Leuchteffizienz von etwa 100 Lumen/Watt eine Leistungsaufnahme von lediglich 0,1 Watt entspricht. Das zweite, stärke Leuchtmittel 4b kann einen maximalen Lichtstrom von ca. 100 - 400 Lumen, bevorzugt etwa 200 Lumen abgeben, was bei gleicher Effizienz wie das Suchlicht 4a einer Leistungsaufnahme von 2 Watt entspricht.

Zum Ein- und Ausschalten des zweiten Leuchtmittels 24b ist in der zylindrischen Mantelfläche des Gehäusemittelteils 25 ein Ein-Aus-Knopf 41 untergebracht. Das als Suchlicht dienende erste Leuchtmittel 4a wird hingegen in dieser Ausführungsform nicht über ein separates Bedienelement aktiviert und deaktiviert, sondern schaltet sich automatisch ein, wenn die Vorrichtung 1 in den Bereitschaftsmodus versetzt wird, was durch mindestens 3s langes Drücken des Programmwahlknopfes 31 erfolgt. Eine Zustandsanzeige in Form farbiger, hinter dem Programmwahlknopf 31 angeordneter LEDs zeigen durch Leuchten in grüner Farbe an, dass sich die Vorrichtung 1 im Bereitschaftszustand befindet. Kurzes (d.h. weniger als 0,5s) ein- oder mehrmaliges kurzes Drücken des Programmwahlknopfes wählt eines der im internen Programmspeicher der Steuerelektronik hinterlegten Programme aus, wobei die Anzahl der Tastendrücke der Nummer des ausgewählten Programms entspricht. Die Zustandsanzeige wechselt spätestens bei der Programmaktivierung, also -ausführung, bevorzugt aber unmittelbar nach der Programmauswahl die Farbe, um dem Benutzer eine Rückmeldung über das gewählte Programm zu geben. Eine bevorzugte mögliche Farbkodierung ist Blau für Programm Nr. 1, Weiß für Programm Nr. 2 und Violett für Programm Nr. 3.

Die Behandlungsdauer beträgt bei den Programmen Nr. 1 und 2 bevorzugt jeweils 3 s, bei Programm Nr. 3 bevorzugt 6 s. Die Behandlungstemperatur beträgt bei den Programmen Nr. 2 und 3 bevorzugt jeweils 51°C, bei Programm Nr. 1 bevorzugt nur 48°C. Damit eignet sich Programm Nr. 1 zur Wärmebehandlung besonders sensibler Hautpartien und/oder Personen, beispielsweise Kinder, Programm Nr. 3 zur wirksamen Behandlung größerer, stärker juckender und/oder geschwollener Stiche und an/bei weniger sensiblen Hautstellen bzw. Personen und Programm Nr. 2 zur Behandlung Stiche durchschnittlicher Größe an normal empfindlicher Haut bzw. bei normal empfindlichen Personen.

In manchen Ausführungsformen der Erfindung erfolgt der Farbwechsel der Zustandsanzeige direkt nach der Programmwahl, das ausgewählte Programm wird aber erst nach einer kurzen Einschaltverzögerung aktiviert. Dies erlaubt es dem Benutzer, der bemerkt, dass er nicht das gewünschte Programm gewählt hat, seine Wahl noch zu ändern. Die Einschaltverzögerung kann zwischen 0,5 bis 4 s betragen, beträgt bevorzugt aber ca. 1 bis 2, beispielsweise 1,5 s. Das Hauptlicht 4b emittiert bevorzugt weißes Licht, das Suchlicht 4a kann weißes oder farbiges, etwa blaues, grünes oder für die Anwendung im Dunkeln hilfreich, rotes Licht emittieren.

Zum Laden der im Gehäuseinneren untergebrachten Batterie ist in dem von der Kappe 26 verdeckten Seite des Gehäuses die USB-C-kompatible Ladebuchse 56 vorhanden, welche in den Figuren 1B und 1D zu sehen ist.

Das kompakte, gedrungene aber dennoch ergonomisch geformte Gehäuse 2 mit dem zylindrischen Mittelteil 25 und den bei aufgesetzter Kappe 26 zwei halbkugeligen Endpartien 22 und 28 ist bevorzugt 65 mm lang und hat einen Durchmesser von 25 mm.

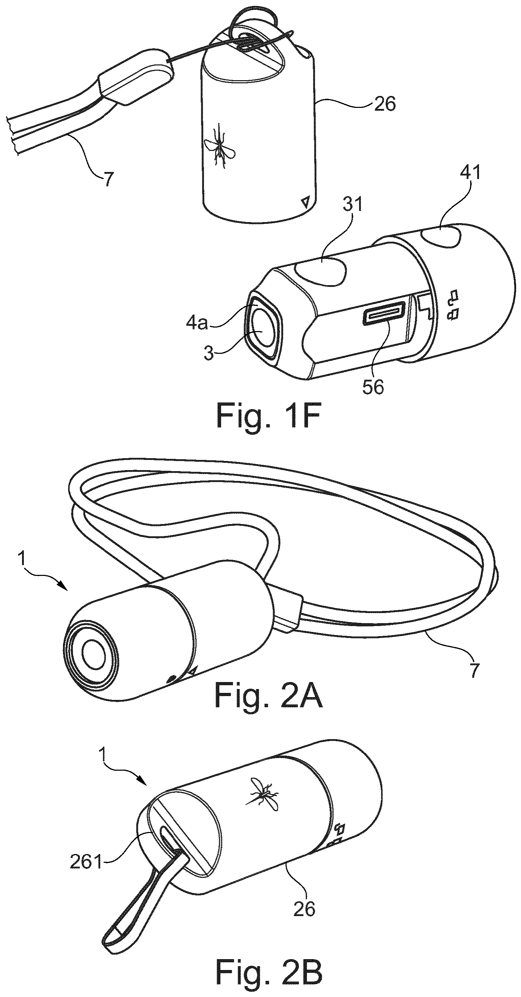

Am oberen, geschlossenen Ende der Kappe 26 befindet sich eine Befestigungsöse 261. In den **Figuren 2A** und **2B** sind zwei Nutzungsmöglichkeiten dieser Öse 261 zum Anschließen von Trage- und Befestigungsmitteln gezeigt. In Figur 2A (wie auch Figur 1F) ist in bekannter Weise mittels eines Einhängeknotens 71 eine Tragekordel 7 angebracht, mit der ein Benutzer die Vorrichtung umhängen oder an einem Haken oder anderen Vorsprung aufhängen kann.

Die **Figur 2B** zeigt hingegen eine Verwendung zur Befestigung eines Karabinerhakens 8, der wiederum zum Einhaken an einer weiteren Trage- oder Befestigungsmöglichkeit variabler Größe genutzt werden kann.

Durch die vorteilhafte Kombination einer Wärmebehandlungsvorrichtung mit Suchlicht und einer Taschenlampe in einem kompakten Gehäuse mit verschiedenen Befestigungsmöglichkeiten ist eine vielseitig verwendbare Vorrichtung geschaffen, die leicht verstaubar und bei Bedarf schnell zu Hand ist.

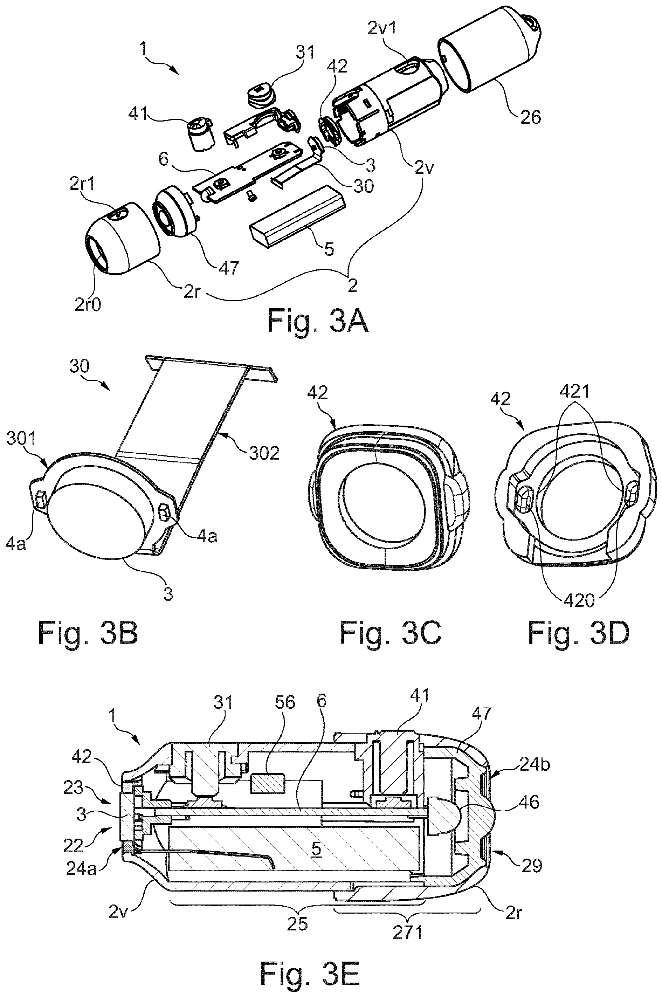

Die Figuren 3A - 3F illustrieren das Innenleben der erfindungsgemäßen Vorrichtung in einer bevorzugten Ausführungsform.

Die einzelnen Komponenten und ihre relative Anordnung sind am besten aus der Explosionsdarstellung der Figur 3A ersichtlich. Danach umfasst das Gehäuse 2 der Vorrichtung 1 einen Gehäusevorderteil 2v und einen Gehäuserückteil 2r, welche zum Verbinden ineinanderschiebbar und über eine Schnappverbindung aneinander fixierbar sind.

Der Gehäuserückteil 2r hat eine stirnseitiges Fenster 2r0 für das als Hauptlicht dienende LED 4b und seitliche Öffnung 2r1 zur Aufnahme des Bedienknopfes 41 zum An- und Ausschalten des Hauptlichts. Die LED 4b ist an der Stirnseite der die Steuerelektronik sowie die eigentlichen Schaltelemente der Bedienelemente beherbergenden Platine 6 untergebracht. Zwischen der LED 4b und dem Fenster 2r0 ist das Linsenelement 7 eingesetzt, welches den Lichtkegel des von der LED 4b emittierten Lichts fokussiert.

Das Gehäusevorderteil 2v umfasst eine vorderseitige Öffnung für die Heizplatte 3, welche auf dem Heizplattenträgerelement 30 untergebracht ist, und eine seitliche Öffnung zur Aufnahme des Programmwahltasters 31.

Das Heizplattenträgerelement 30 ist in der **Figur 3B** im Detail dargestellt. Es umfasst einen Trägerabschnitt 301 und einen abgewinkelten Kontaktabschnitt 302, der als Kontaktschiene zu einem Pol des Energiespeichers 5 dient. Auf dem Heizplattenträgerelement sind zu beiden Seiten der Heizplatte zwei LEDs 4a als Leuchtmittel angeordnet, welche zusammen die Suchlichtfunktion erfüllen um im Dunkeln eine juckende Hautstelle leicht auffinden und gezielt behandeln zu können, als dies durch Tasten allein möglich wäre.

Die **Figuren 3C** und **3D** zeigen die Abdeckung 42 mit den zwei rückwärtigen Aufnahmen 420 für die beiden LEDs 4a. Durch die Teilbereiche 421 der Abdeckung werden die LEDs 4a von der Wärmestrahlung der Heizplatte 3 abgeschirmt.

Wie in der Figur 3A andeutungsweise und im Längsschnitt der **Figur 3E** am besten zu erkennen ist, erstreckt sich die Platine 6 von einem Ende des Innenraums 20 des Gehäuses 2 zum anderen und liegt in einer Mittenebene des Gehäuses. Dadurch können die stirnseitig der Platine angeordneten Komponenten Hauptlicht-LED 4b und Heizplatte 3 mit nebenbei angeordneten Suchlicht-LEDs 4a vorteilhaft einfach von der Platine 6 aus kontaktiert bzw., im Fall der Hauptlicht-LED 4b unmittelbar auf dieser angebracht werden. Zudem verbleibt der Rückwärtige Halbraum 20b des Gehäuseinnenraums für den Energiespeicher 5, der diesen Halbraum 20b nahezu ausfüllt und so die vorhandene elektrische Energie maximiert.

### Bezugszeichenliste

- 1: multifunktionale Wärmebehandlungsvorrichtung
- 2: Gehäuse
- 2v: Gehäusevorderteil
- 2v0: Öffnung für 3 in 2v
- 2v1: Öffnung für 31 in 2v
- 2r: Gehäuserückteil
- 2r0: Fenster für 4b in 2r
- 2r0: Öffnung für 41 in 2r
- 20: Kerbe zur Bajonettverriegelung von 26 an 2
- 21: vorderes Ende von 2
- 22: vorderer Endbereich von 2
- 23: Behandlungsfläche
- 24: Leuchtmittelbereich
- 24a: Leuchtmittelbereich für 4a
- 24b: Leuchtmittelbereich für 4b
- 25: Mittelteil von 2
- 26: Kappe
- 261: Befestigungsöse
- 27: Hauptteil
- 271: Griffteil
- 28: hinterer Endbereich von 2
- 29: hinteres Ende von 2
- 3: Heizplatte
- 31: Programmwahlknopf
- 4: Leuchtmittel
- 4a: Suchlicht-LED
- 4b: Hauptlicht-LED
- 41: Ein-/Aus-Knopf für 4b
- 42: Abdeckung für 4a
- 420: Ausnehmung in 42 für 4a
- 421: Separationsabschnitt
- 47: Linseneinsatz
- 5: Energiespeicher
- 56: Ladebuchse
- 6: Platine
- 7: Tragekordel
- 8: Karabinerhaken

## Patentansprüche

1. Multifunktionale Vorrichtung, unter anderem zur Behandlung von Insektenstichen und -bissen, umfassend:
- ein Gehäuse (2),
- eine Heizplatte, die in einer an einem vorderen Ende (21) des Gehäuses (2) liegenden Behandlungsfläche (23) angeordnet ist,
- ein oder mehrere Leuchtmittel (4, 4a, 4b), welche/s in einem oder mehreren Leuchtmittelbereichen (24, 24a, 24b) des Gehäuses (2) angeordnet ist,
- eine elektrische Energiequelle (5), insbesonder ein elektrischer Energiespeicher (5), mittels der die Heizplatte (3) und das Leuchtmittel (4) mit elektrischer Energie versorgbar ist, und
- eine Steuerelektronik, welche dazu vorbereitet ist, die Heizplatte (3) mit der Energiequelle (5) entnommener elektrischer Energie zu versorgen, und die Heizplatte (3) dabei für eine gewünschte Behandlungsdauer auf eine gewünschte Behandlungstemperatur aufzuheizen,
**dadurch gekennzeichnet, dass**
die Steuerelektronik auf einer Platine (6) untergebracht ist, welche in einer Mittenebene des Gehäuses angeordnet ist.

2. Vorrichtung nach Anspruch 1, wobei das Leuchtmittel (4, 4b) an einer Stirnseite der Platine (6) angeordnet ist und eine Lichtemissionsrichtung des Leuchtmittels (4, 4b) insbesondere von der Platine (6) weg weist.

3. Vorrichtung nach dem vorhergehenden Anspruch, wobei die elektrische Energiequelle (5) den zwischen der Platine (6) und einer Innenwandung des Gehäuses (2) verbleibenden, insbesondere im Wesentlichen halbzylindrischen, Raum auf einer Seite der Platine (6) nahezu vollständig ausfüllt.

4. Vorrichtung nach einem der vorhergehenden Ansprüche, wobei ein erster Leuchtmittelbereich (24a) der ein oder mehr Leuchtmittelbereiche (24, 24a, 24b) in dem vorderen Ende (21) angeordnet ist, und insbesondere die Behandlungsfläche (23) ringförmig umgibt und der erste Leuchtmittelbereich (24a) ein erstes Leuchtmittel (4a) der ein oder mehr Leuchtmittel (4, 4a, 4b) aufweist, welches insbesondere einen maximalen Lichtstrom von 1 bis 100 Lumen, bevorzugt zwischen 5 und 40 Lumen hat und weiterhin bevorzugt das erste Leuchtmittel (4a) weißes Licht oder farbiges Licht emittiert, insbesondere rotes, blaues oder grünes Licht.

5. Vorrichtung nach dem vorhergehenden Anspruch, wobei das erste Leuchtmittel (4, 4a) und die Heizplatte (3) nebeneinander auf einem, insbesondere von der Platine (6) separaten, Heizplattenträgerelement (30) untergebracht sind, wobei insbesondere zwischen Heizplatte (3) und Heizplattenträgerelement (30) mit Ausnahme der elektrischen Zuleitungen zur Heizplatte (3) ein Luftspalt verbleibt, so dass nahezu keine Kontaktwärmeübertragung von der Heizplatte (3) auf das Heizplattenträgerelement (30) stattfindet, wobei weiterhin bevorzugt das erste Leuchtmittel (4a) in einem Abstand zur Heizplatte (3) auf dem Heizplattenträgerelement (30) angeordnet ist, welcher in etwa seinen Abmessungen entspricht, wobei der Abstand insbesondere 0,5 - 2 mm beträgt.

6. Vorrichtung nach dem vorhergehenden Anspruch, wobei sie eine lichtdurchlässige Abdeckung (42) für das erste Leuchtmittel (4, 4a) umfasst, wobei das erste Leuchtmittel in eine rückwärtige Ausnehmung (420) der Abdeckung aufgenommen ist, und ein Teilbereich (421) der Abdeckung zwischen dem ersten Leuchtmittel (4, 4a) und der Heizplatte liegt, so dass keine direkte Sichtlinie zwischen dem Leuchtmittel (4, 4a) und der Heizplatte besteht.

7. Vorrichtung nach einem der vorhergehenden Ansprüche, wobei das Leuchtmittel (4) unabhängig von der Heizplatte (3) betreibbar ist, insbesondere das Leuchtmittel (4) und die Heizplatte (3) über separate Bedienmittel (31, 41) unabhängig voneinander aktivierbar sind.

8. Vorrichtung nach einem der vorhergehenden Ansprüche, wobei ein zweiter Leuchtmittelbereich (24b) der ein oder mehr Leuchtmittelbereiche (24, 24a, 24b) an einem dem vorderen Ende (21) gegenüberliegenden hinteren Ende (29) des Gehäuses (2) angeordnet ist und der zweite Leuchtmittelbereich (24, 24b) ein zweites Leuchtmittel (4b) der ein oder mehr Leuchtmittel (4, 4a, 4b) umfasst, wobei das zweite Leuchtmittel insbesondere einen maximalen Lichtstrom von zwischen 50 und 1000 Lumen, bevorzugt zwischen 100 und 400 Lumen hat und weiterhin bevorzugt das zweite Leuchtmittel (4b) weißes Licht emittiert.

9. Vorrichtung nach einem der vorhergehenden Ansprüche, wobei sie ein Bedienelement (41) umfasst, insbesondere einen Knopf oder einen Schalter, mittel dessen das erste Leuchtmittel (4a) und/oder das zweite Leuchtmittel (4b) ein- und ausschaltbar ist, insbesondere ein weiteres Bedienelement für jedes der Leuchtmittel (4, 4a, 4b), so dass jedes der Leuchtmittel (4, 4a, 4b) unabhängig vom Betriebszustand der Vorrichtung aktivierbar und deaktivierbar ist.

10. Vorrichtung nach einem der vorhergehenden Ansprüche, wobei sie eine Kappe (26) umfasst, welche das vordere Gehäuseende (21) bedeckt, wobei die Kappe (26) bevorzugt mittels eines Bajonettverschlusses an dem Gehäuse (2) verriegelbar und/oder verriegelt ist.

11. Vorrichtung nach einem der vorhergehenden Ansprüche, wobei die elektrische Energiequelle (5) ein Energiespeicher ist, insbesondere eine Batterie, bevorzugt eine wiederaufladbare Batterie wie beispielsweise eine Lithium-Ionen Batterie, welche entweder aus der Vorrichtung (1) entnehmbar ist oder die Vorrichtung (1) umfasst weiterhin eine Ladebuchse (56), mittels der bei Anschluss einer geeigneten Spannungsquelle der Energiespeicher (5) aufladbar ist, wobei die Ladebuchse (56) insbesondere in einer von dem vorderen Gehäuseende (21) und/oder dem hinteren Gehäuseende (29) verschiedenen Seite des Gehäuses (2) angeordnet ist.

12. Vorrichtung nach einem der vorhergehenden Ansprüche, wobei das Gehäuse (2):
- länglich ist, und insbesondere einen zylindrischen Mittelteil (25) und in etwa halbkugelige Endbereiche (22), (28) aufweist, und/oder
- gegebenenfalls mit aufgesetzter Kappe, eine Länge von zwischen 50 und 100 mm, insbesondere ca. 65 mm und einen Durchmesser von zwischen 20 und 40, insbesondere ca. 25 mm hat, wobei die Kappe (26) insbesondere eine Höhe von zwischen 1/3 und 4/5, insbesondere von circa 2/3 der Gehäuselänge aufweist, und/oder
- spritzwassergeschützt oder wasserdicht ist.

13. Vorrichtung nach einem der vorhergehenden Ansprüche, wobei die Behandlungsdauer zwischen 2 Sekunden und 10 Sekunden, insbesondere zwischen 3 Sekunden und 6 Sekunden beträgt, und die Behandlungstemperatur zwischen 46 Grad Celsius und 54 Grad Celsius, insbesondere zwischen 48 Grad Celsius und 51 Grad Celsius liegt, besonders bevorzugt beträgt die Behandlungsdauer 3 Sekunden bei einer Behandlungstemperatur von 48 Grad Celsius oder 51 Grad Celsius und/oder die Behandlungsdauer beträgt 6 Sekunden bei einer Behandlungstemperatur von 51 Grad Celsius.

14. Vorrichtung nach einem der vorhergehenden Ansprüche, wobei die Steuerelektronik einen Programmspeicher umfasst, in dem zwei oder mehr Behandlungsprogramme abgespeichert sind und die Vorrichtung (1) weiterhin ein Bedienmittel aufweist, insbesondere einen Programmwahlknopf oder - taster (31), mittels dessen durch einen Benutzer der Vorrichtung (1) eines der im Programmspeicher abgespeicherten Programme auswählbar und/oder ausführbar ist, wobei bevorzugt ein Programm nach dem Auswählen automatisch ausgeführt wird, wobei insbesondere die im Programmspeicher abgespeicherten Behandlungsprogramme zumindest umfassen:
- ein erstes Behandlungsprogramm mit einer Behandlungsdauer von 3 Sekunden und einer Behandlungstemperatur von 48 Grad Celsius, und/oder
- ein zweites Behandlungsprogramm mit einer Behandlungsdauer von 3 Sekunden und eine Behandlungstemperatur von 51 Grad Celsius, und/oder
- ein drittes Behandlungsprogramm mit einer Behandlungsdauer von 6 Sekunden und einer Behandlungstemperatur von 51 Grad Celsius.

15. Vorrichtung nach einem der beiden vorhergehenden Ansprüche wobei sie weiterhin eine An-/Austaste (31) umfasst, die bevorzugt unterhalb der Kappe (26) angeordnet ist, und dazu vorbereitet ist:
- von einem ausgeschalteten Zustand in einen Bereitschaftszustand überzugehen, wenn die An-/Austaste (31) für mindestens eine Aktivierungszeitdauer, insbesondere zwischen 2 Sekunden und 4 Sekunden, bevorzugt 3 Sekunden, gedrückt wird, und
- aus dem Bereitschaftszustand in einen aktiven Zustand, in dem die Heizplatte gemäß einem der abgespeicherten Programme aktiviert ist, überzugehen, wenn die An-/Austaste (31) ein oder mehrmals kurz, d.h. für eine Sekunde oder weniger, gedrückt wird, wobei insbesondere eine Anzahl der An-/Aus-Tastendrücke entscheidet, welches der mindestens zwei abgespeicherten Behandlungsprogramme ausgeführt wird,
- wobei die Steuerelektronik (31) insbesondere dazu vorbereitet ist, dass erste Leuchtmittel (4a) zu aktivieren, wenn die Vorrichtung (1) im Bereitschaftszustand und/oder im aktiven Zustand ist,
wobei die Vorrichtung insbesondere zum Bereitstellen einer Rückmeldung über den derzeitigen Betriebszustand und/oder das derzeit auswählte und/oder ausgeführte Programm eine Zustandsanzeige umfasst, wobei die Zustandsanzeige insbesondere ein hinter der An-/Austaste (31) angeordnetes drittes Leuchtmittel ist, welches im ausgestalteten Zustand der Vorrichtung (1) nicht leuchtet und im Bereitschaftszustand und im aktiven Zustand leuchtet, wobei insbesondere das dritte Leuchtmittel farbig, insbesondere eine farbige LED ist, und vorbereitet ist,
- in einer ersten Farbe zu leuchten, insbesondere in Grün, wenn sich die Vorrichtung (1) im Bereitschaftszustand befindet, in einer von der ersten Farbe verschiedenen zweiten Farbe leuchten, wenn sich die Vorrichtung (1) im aktiven Zustand befindet, in dem die Heizplatte (3) beheizt ist,
wobei die zweite Farbe:
- Blau ist im Falle des ersten Behandlungsprogramms, und
- Weiß ist im Falle des zweiten Behandlungsprogramms,
- Violett ist im Falle des dritten Behandlungsprogramms.
